# EUROPÄISCHE PATENTSCHRIFT

(11) **EP 0 316 378 B1**
(45) Veröffentlichungstag und Bekanntmachung des Hinweises auf die Patenterteilung: **02.11.1994**
(21) Anmeldenummer: 88904223.0
(22) Anmeldetag: 19.05.1988
(51) Int. Cl.: C12N 15/70, C12P 21/00

(54) **EXPRESSIONSVEKTOR ZUR REGULIERBAREN EXPRESSION VON FREMDGENEN IN PROKARYONTEN**
EXPRESSION VECTOR FOR ADJUSTABLE EXPRESSION OF EXOGENOUS GENES IN PROKARYOTES
VECTEUR D'EXPRESSION POUR L'EXPRESSION REGULABLE DE GENES ETRANGERS DANS DES PROCARYOTES

(30) Priorität: 20.05.1987 DE 3716957
(43) Veröffentlichungstag der Anmeldung: 24.05.1989
(73) Patentinhaber: BOEHRINGER MANNHEIM GMBH, 68298 Mannheim (DE)
(72) Erfinder: SCHUMACHER, Günther, D-8139 Bernried (DE); JARSCH, Michael, D-8000 München 70 (DE); BOOS, Winfried, D-7750 Konstanz (DE)
(74) Vertreter: Huber, Bernhard, Dipl.-Chem.
(86) Internationale Anmeldenummer: EP8800446
(87) Internationale Veröffentlichungsnummer: WO8809373

(56) Entgegenhaltungen:
- EP-A- 0 035 384
- EP-A- 0 137 633
- EP-A- 0 141 362
- EP-A- 0 177 343
- EP-A- 0 215 388
- WO-A-84/04755
- WO-A-86/04356
- US-A- 4 595 658
- Journal of Bacteriology, Band 163, no. 1, July 1985, American Society for Microbiology, N. Müller et al., pp. 37-45
- Mol. Gen. Genet. Band 208, 1987, Springer-Verlag, A. Scholle et al., pp. 247-253
- The Journal of Biological Chemistry, Band 258, 25. September 1983 (US), J. Benjamin Scripture et al., pp. 10853-10855
- Gene, Band 51, Nrn. 2-3, 1987, Elsevier Science Publishers B.V. (Biomedical Division), (Amsterdam, NL), M.J.R. Stark, pp. 255-267
- Chemical Abstracts, Band 95, 1981, (Columbus, Ohio, US), K. Ito et al., p. 333, abstract 57836t
- Journal of Bacteriology, Band 153, no. 1, January 1983, American Society for Microbiology, S. Harayama et al., pp. 408-415
- The Journal of Biological Chemistry, Band 257, no. 15, 10 August 1982, (US), B. Rotman et al., pp. 9030-9034

## Beschreibung

Die Erfindung betrifft einen Expressionsvektor zur regulierbaren Expression von Fremdgenen in Prokaryonten.

Zur Expression von Proteinen in Prokaryonten benötigt man Expressionsvektoren. Derartige Vektoren müssen außer dem Gen des zu exprimierenden Proteins noch Regulationssequenzen enthalten, welche die Transkriptions- und Translationsprozesse in der zur Proteinproduktion verwendeten Zelle ermöglichen. Derartige Regulationssequenzen stammen - sofern in Prokaryonten exprimiert werden soll - aus Prokaryonten und enthalten beispielsweise Sequenzen von Promotoren, Operatoren und ribosomalen Bindungsstellen. Ublicherweise werden derartige Regulationssequenzen in einem Vektor, z. B. dem Plasmid pBR322 und dessen Derivaten, dem Gen vorgeschaltet. Diese Vektoren enthalten dann neben den vorher beschriebenen Regulationssequenzen auch noch einen Replikationsursprung und einen Marker zur Selektion des Plasmids, z. B. Tetrazyklinresistenz, Ampicillinresistenz, Canamycinresistenz und andere.

Die DNA-Einheit, die von der DNA-abhängigen RNA-Polymerase erkannt und in Messenger-RNA übersetzt wird, bezeichnet man als Transkriptionseinheit. Eine solche Transkriptionseinheit besteht aus Erkennungssequenzen für die DNA-abhängige RNA-Polymerase, Erkennungssequenzen für die Initiation der ribosomen Funktion (Shine-Dalgarno-Sequenz), dem Startkodon ATG, einem Stopkodon und Terminationssequenzen, an denen die Transkription beendet wird. Bestimmte Gene, nämlich solche, deren Genprodukte nicht im Cytoplasma lokalisiert bleiben, sondern in das Periplasma bzw. in die äußere Membran sekretiert werden, können daneben noch andere Erkennungssequenzen enthalten. Eine solche Erkennungssequenz zur Exkretion von Proteinen nennt man Signalsequenz. Eine Signalsequenz enthält charakteristische geladene Segmente, hydrophobe Bereiche und hydrophile Bereiche und eine Signalsequenz-Spaltstelle (siehe Übersichtsartikel Mechanismus of Protein Localisation, Microbiol. Reviews 1983, 47. Seite 314-344).

An einen Expressionsvektor werden demnach folgende Anforderungen gestellt:
a) er muß einen starken Promotor enthalten,
c) Fusionen mit Fremdgenen sollten leicht durchführbar sein und
d) der Promotor incl. eines anschließenden Fremdgenabschnitts sollte geeignet sein, eine Lokalisation des Proteins sowohl im Cytoplasma als auch im Periplasma bzw. im Medium zu ermöglichen.

Es ist bekannt, daß der Umfang der Expression des Fremdgens wesentlich vom Promotor abhängt. Um zu beurteilen, ob ein bestimmter Promotor für die Expression von Fremdgenen besonders geeignet ist, kommt es allerdings nicht alleine auf die Menge des pro Zelle produzierten Fremdproteins an, sondern sehr oft ist es auch wünschenswert, daß das Genprodukt nicht während der gesamten Wachstumsphase, sondern nur über einen bestimmten Zeitraum - vorzugsweise der späten Wachstumsphase - synthetisiert wird. Dies ist vor allen Dingen bei der Expression von Genprodukten, die, wenn sie in großer Menge vorhanden sind, entweder für die Zellen toxisch sind oder das Wachstum der Zellen hemmen, wünschenswert. Daher ist es oft erforderlich, die Aktivität eines Promotors zu Beginn der Fermentationsphase zu unterdrücken, so daß zunächst eine umfangreiche Biomasse produziert wird. Anschließend sollte durch geeignete Maßnahmen der Promotor stimuliert werden und die Expression des Fremdgens erfolgen können.

Zu diesem Zwecke bekannte Promotoren sind beispielsweise der lac-Promotor, der trp-Promotor und der λ-P_{L}-Promotor. Für eine großtechnische Produktion von heterologen Proteinen sind diese Promotoren jedoch nicht gut geeignet. Für den lac-Promotor ist bekannt, daß dieser durch Glucose abgeschaltet wird, jedoch nicht vollständig genug, um die Synthese von "toxischen" Proteinen zu verhindern. Auch der trp-Promotor ist kein für die großtechnische Produktion besonders gut geeigneter Promotor. Abgesehen davon, daß die Verwendung von hohen Tryptophankonzentrationen zur Repression die Fermentation wesentlich verteuert, hat sich gezeigt, daß Tryptophan keine vollständige Repression ermöglicht, so daß auch hier während der Anfangsphase der Fermentation eine störende Expression erfolgen kann. Auch der λ-P_{L}-Promotor ist für eine großtechnische Fermentation nicht geeignet. Der Repressionsmechanismus erfolgt hier durch die Bindung eines thermolabilen Repressors an einen hinter dem Promotor befindlichen Operator bei 32°C. Durch Temperaturerhöhung auf 42°C wird der Repressor inaktiviert, wodurch die Transkription ermöglicht wird. Für eine Großproduktion, welche mit Fermentationsvolumina von 50 oder 100 m³ verbunden ist, ist eine derartige Temperaturerhöhung mit großen Schwierigkeiten verbunden. Darüberhinaus hat sich gezeigt, daß die Induktion des λ-P_{L}-Promotors in einer frühen Wachstumsphase erfolgen muß, so daß die für eine biotechnologische Großproduktion notwendige Biomasse nicht erreicht werden kann.

Müller et al. (J. Bacteriol. 163 (1985), 37-45) beschreiben die Charakterisierung des mgl-Operons von Salmonella typhimurium und seiner Genprodukte. Es werden DNA-Vektoren offenbart, welche als Regulationssequenz die Promotor-Operator-Region und die Initiationsstelle der Translation des mgl-Operons enthalten. Die dort offenbarten Vektoren enthalten jedoch keine Fremdgene. Weiterhin ist in dieser Arbeit auch nicht beschrieben, daß der mgl-Promotor mit Glucose und anderen Katabolyt-reprimierbaren Zuckern nahezu vollständig reprimiert werden kann.

Scripture und Hogg (J. Biol. Chem. 258 (1983), 10853-10855) beschreiben die Nukleotidsequenzen, welche die Signalpeptide des Galactose-bindenden Proteins und des Arabinose-bindenden Proteins definieren. Es werden Plasmide offenbart, die die Promotor-Operator-Region und die Initiationsstelle der Translation des mgl-Operons enthalten. Diese Vektoren enthalten jedoch zusätzlich für das reife mgl-B-Protein kodierende Sequenzen. Auch in dieser Arbeit wird nicht beschrieben, daß der mgl-Promotor durch Glucose und andere Katabolyt-reprimierende Zucker nahezu vollständig reprimierbar ist.

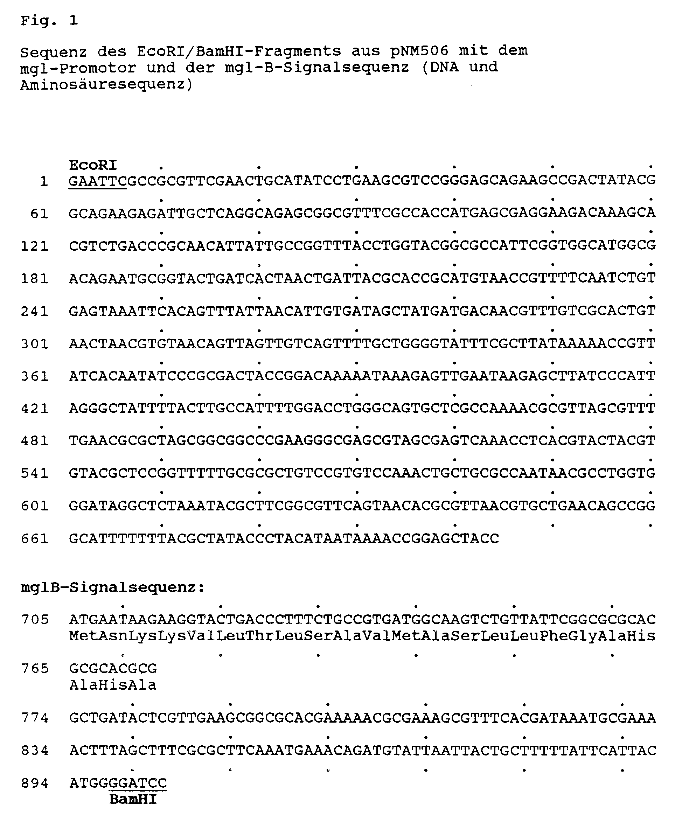

Der Erfindung liegt daher die Aufgabe zugrunde, die vorstehend geschilderten Schwierigkeiten zu beseitigen und einen Expressionsvektor zur regulierbaren Expression von Fremdgenen in Prokaryonten bereitzustellen. Gelöst wird diese Aufgabe erfindungsgemäß durch einen Expressionsvektor zur regulierbaren Expression von Fremdgenen in Prokaryonten, dadurch gekennzeichnet, daß er aus einem mit Glukose und anderen Katabolyt-reprimierenden Zuckern nahezu vollständig reprimierbaren DNA-Vektor besteht, der die Regulationssequenz des mgl-Operons, die aus den Nukleotiden 1 bis 704 der DNA-Sequenz, die in Fig. 1 dargestellt ist, oder einer bei Standardbedingungen damit hybridisierenden, aber noch den gesamten mgl-Promotor enthaltenden Sequenz besteht, und die Initiationsstelle der Translation des mgl-Operons sowie wenigstens ein Fremdgen enthält, welches unter der Expressionskontrolle der mgl-Operon-Regulationssequenz steht, wobei sich zwischen dem Promotor und der Insertionsstelle des Fremdgens keine für das reife mglB-Protein codierenden Sequenzen befinden.

Der im erfindungsgemäßen Expressionsvektor enthaltene mgl-Promotor ist ein Teil des mgl-Operons, welches neben dem Promotor noch 4 Strukturgene, mglA, mglB, mglE und mglC enthält, die der Kontrolle des mgl-Promotors unterliegen. Hierbei kodiert das mglB-Gen für ein Galactosidase-Bindungsprotein von 33.000 Dalton, mglA, mglC und mglE kodieren jeweils für membrangebundene Proteine. Vom mgl-Operon werden somit Proteine exprimiert, die für den Transport von Galactose aus dem Medium in die Bakterienzelle verantwortlich sind.

Zur Herstellung des erfindungsgemäßen Vektors kann das mgl-Operon prinzipiell aus dem Genom einer Zelle, die in der Lage ist, von außen zugeführte Galactose zu verwerten, beispielsweise aus Salmonella typhimurium, DSM 554 oder aus E. coli, MC 4100 (DSM 4090) (J. Biol. Chem. 258 (1983) 10853-10855, J. Bacteriol. 153 (1983) 408-415) isoliert werden.

Die Isolierung des mgl-Operons aus Salmonella typhimurium kann erfindungsgemäß so erfolgen, daß die genomische DNA mit EcoRI gespalten und ein 6,3 kb großes Fragment isoliert wird, welches in üblicher Weise kloniert und anschließend exprimiert werden kann (vgl. hierzu J. Bacteriol. 163, 37-85, 1985, sowie die dort zitierte Literatur). Aus dem in dieser Literaturangabe zitierten Plasmid pNM 506 kann durch Spaltung mit EcoRI und BamHI ein ca. 900 bp großes Fragment herausgespalten und isoliert werden. Von der DNA-Sequenz dieses Fragments wurde unmittelbar nach der EcoRI-Erkennungssequenz beginnend die Sequenz bestimmt (Fig. 1). An Position 705 bis 707 befindet sich das Startkodon ATG. Fünf Nukleotide davor liegt die als Shine-Dalgarno-Sequenz erkennbare Nukleotidabfolge GGAG. Dieses Fragment enthält den mgl-Promotor, der jedoch nur einen Teil dieser DNA-Sequenz darstellt.

Im Gegensatz zu den bisher bekannten anderen Regulationssystemen kann der mgl-Promotor mit Glucose und anderen Katabolyt-reprimierenden Zuckern, wie z. B. Fructose oder Glucose-6-phosphat, nahezu vollständig reprimiert werden. Mit der erfindungsgemäßen Verwendung des mgl-Promotors ist es also möglich, auf besonders einfache Weise die Genexpression zu steuern. Beispielsweise kann bei der Fermentation Glucose als C-Quelle in bestimmter Menge zugegeben werden. Nachdem die Glucose aufgebraucht ist, beginnt die Expression, wobei dann eine andere C-Quelle zugesetzt werden muß, die nicht Katabolit-reprimierend wirkt (z. B. Glycerin oder Succinat). Durch zusätzliche Zugabe von Fucose kann die Promotoraktivität noch weiter gesteigert werden. Die Repression durch Glucose macht etwa einen Faktor 100 aus.

Der DNA-Vektor, der dem erfindungsgemäßen Expressionsvektor zugrundeliegt, kann ein Plasmid, ein Phagengenom oder auch ein Shuttle-Vektor, der zur Expression in grampositiven oder gramnegativen Bakterien, insbesondere in Enterobakterien, geeignet ist, sein. Als Expressionsvektoren sind die in den genannten Bakterien vermehrbaren Vektoren geeignet. Beispiele sind pBR-Vektoren wie pBR322, pUC-Vektoren wie pUC18 sowie die Phagen Lambda und M13 und ihre Derivate. Bevorzugt enthält der Expressionsvektor zur Insertion des zu exprimierenden Fremdgens einen Polylinker. Solch ein Polylinker wird nach bekannten Methoden hinter den Regulationssequenzen in den Vektor eingebaut. Vorzugsweise wird ein Polylinker verwendet, der eine oder mehrere Restriktionsschnittstellen enthält, die im verwendeten Plasmid oder im Phagengenom nicht oder selten vorkommen. Mit dem oder den entsprechenden Restriktionsenzymen wird der Vektor sodann geschnitten und das Fremdgen, welches entweder mit der gleichen Restriktionsendonuklease geschnitten ist oder dessen beide Enden in bekannter Weise so verändert wurden, daß sie ebenfalls zu der fraglichen Schnittstelle passen, einligiert.

Um die spätere Lokalisation des Genprodukts in der Wirtszelle von vorneherein zu bestimmen, kann zwischen die Regulationssequenzen und das Fremdgen die DNA-Sequenz für ein Signalpeptid zwischengeschaltet werden. Bevorzugt wird erfindungsgemäß die DNA-Sequenz für das Signalpeptid des mglB-Proteins, welches eine Lokalisation im Periplasma bewirkt, verwendet (J. Biol. Chem. 258 (1983) 10853-10855). An seiner Stelle kann aber auch die DNA-Sequenz einer anderen bekannten Signalsequenz oder einer Konsensussequenz (Nature 321 (1986), 706-708) verwendet werden. Solche Konsensussequenzen können durch Vergleich von bekannten Signalsequenzen untereinander ermittelt werden. Ohne Signalpeptid verbleibt das Fremdgenprodukt im Cytoplasma.

Gemäß einer ersten Ausführungsform der Erfindung enthält ein erfindungsgemäßer Expressionsvektor vorzugsweise die Sequenz, welche in Fig. 1 dargestellt ist und den mgl-Promotor umfaßt, oder eine Sequenz, die bei Standardbedingungen damit hybridisiert. Dies kann auch eine im Vergleich zu der Sequenz der Fig. 1 verkürzte DNA-Sequenz sein, die aber noch den gesamten mgl-Promotor enthalten muß. Unter Standardbedingungen sind Hybridisierungsbedingungen zu verstehen, wie sie in T. Maniatis, Molecular Cloning CSH (1982) 383-389 beschrieben sind.

Das Expressionsprodukt eines anschließenden Fremdgens ist dann im Cytoplasma lokalisiert. Wird eine Exkretion ins Periplasma gewünscht, so wird gemäß einer anderen bevorzugten Ausführungsform die mgl-B-Signalsequenz, die im unteren Teil der Fig. 1 dargestellt ist (Signalsequenz des mglB-Proteins) an die vorherige Sequenz angeschlossen.

Weitere Gegenstände der Erfindung sind die Plasmide M13mgl506 und M13mglEcok, die eine ein ca. 900 bp EcoRI/BamHI-Fragment des Plasmids pNM506 (Fig. 1) entsprechende DNA-Sequenz sowie im Falle von M13mglEcoK zusätzlich eine Polylinkersequenz (4x EcoK-Cassette, Nucleic Acids Research 13, (1985), 8561-71) insertiert in die doppelsträngige, replikative Form des Phagen M13mp18 (Sequenz in Gene 33 (1985), 103-119 beschrieben) enthalten. Erfindungsgemäß wird das Plasmid M13mgl506 hergestellt, indem man eine dem EcoRI/BamHI-Fragment des Plasmids pNM506 entsprechende DNA-Sequenz in die ebenfalls mit EcoRI und BamHI geschnittene doppelsträngige replikative Form des Phagen M13mp18 einligiert. Das erfindungsgemäße Verfahren zur Herstellung des Plasmids M13mglEcoK beinhaltet das Einligieren einer dem EcoRI/BamHI-Fragment des Plasmids pNM506 entsprechenden DNA-Sequenz zusammen mit einer dem BamHI/EcoRI-Fragment aus dem Plasmid M13K11 entsprechenden DNA-Sequenz, die vier EcoK-Schnittstellen enthält (4x EcoK-Cassette, Nucleic Acids Research 13, (1985), 8561-71), welche die Durchführung einer Deletionsmutagenese erleichtert, bei der ein Fremdgen direkt hinter die Operonsequenz eingebracht werden kann (Methods Enzymol. 100 (1983), 468-500; Nucleic Acids Res. 10 (1982), 6487-6500) und als Polylinker zur Insertion eines beliebigen Fremdgens fungieren kann, in die mit EcoRI gespaltene replikative Form des Phagen M13mp18.

Die erfindungsgemäße Verwendung einer Regulationssequenz des mgl-Operons ermöglicht die durch Katalyt-reprimierende Zucker, wie z. B. Glucose, regulierbare Expression von Fremdgenen, wodurch es möglich wird, auch im großtechnischen Umfang sogar für die exprimierende Zelle toxische Genprodukte herzustellen, indem die Expression nur im späten Wachstumszyklus nach Entfernung oder Fermentation des Zuckers ermöglicht wird. Es ist dabei auch möglich, die spätere Lokalisation des Genprodukts im voraus zu bestimmen, wobei eine Lokalisation im Periplasma oder sogar Abgabe des Genprodukts ins Medium durch den oben erläuterten Aufbau des erfindungsgemäßen Expressionsvektor vorherbestimmt werden kann, z. B. durch Verwendung eines Prokaryonten, der Substanzen ins Medium abgeben kann, beispielsweise grampositive Bakterien oder E. coli Mutanten, wie sie z. B. in FEMS Microbiol. Lett. 5I (1979) 411-416 oder J. Bact. 145 (1981) 1351-1358 beschrieben sind. Dadurch wird die Notwendigkeit eines Zellaufschlusses vermieden und ermöglicht, bei für die Wirtszelle nicht toxischen Genprodukten die Produktion fortlaufend weiterzuführen, wobei das Produkt laufend aus dem Medium gewonnen werden kann.

### Beispiel 1

### Isolierung der mgl-Promotor-Operratorregion.

Aus dem Plasmid pNM506 (J. Bacteriol. 163 (1985), 37-45) wird durch Spaltung mit EcoRI und BamHI ein 897 BasenPaar großes Fragment gewonnen (Fig. 1).

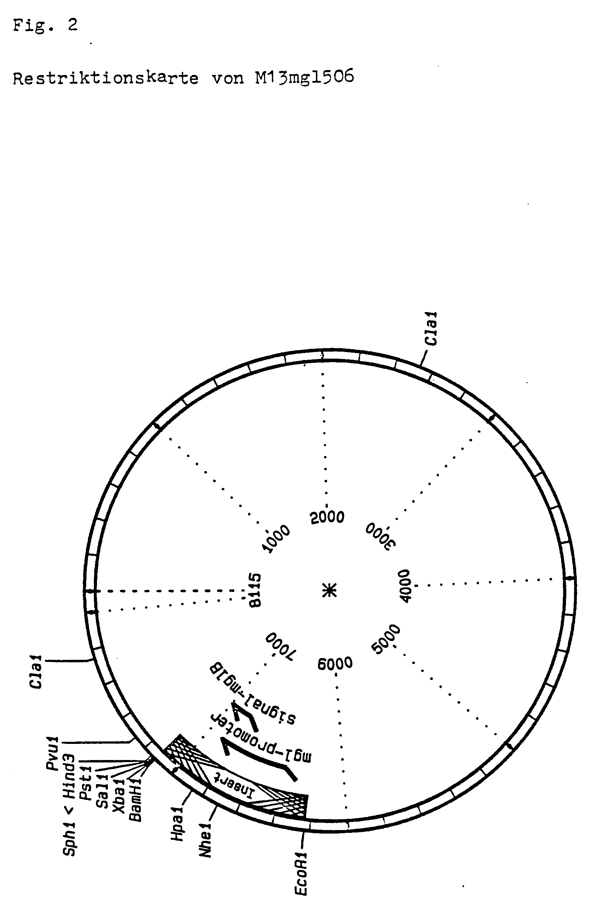

Dieses Fragment enthält die Promotor-Operator-Region des mgl-Operons. Es wird in die doppelsträngige replikative Form des Phagen M13mp18 (Sequenz vgl. Gene 33 (1985) 103-119), die ebenfalls mit EcoRI und BamHI gespalten wurde mit Hilfe von T4 DNA-Ligase einligiert (Fig. 2). Das entstehende Plasmid trägt die Bezeichnung M13mgl506.

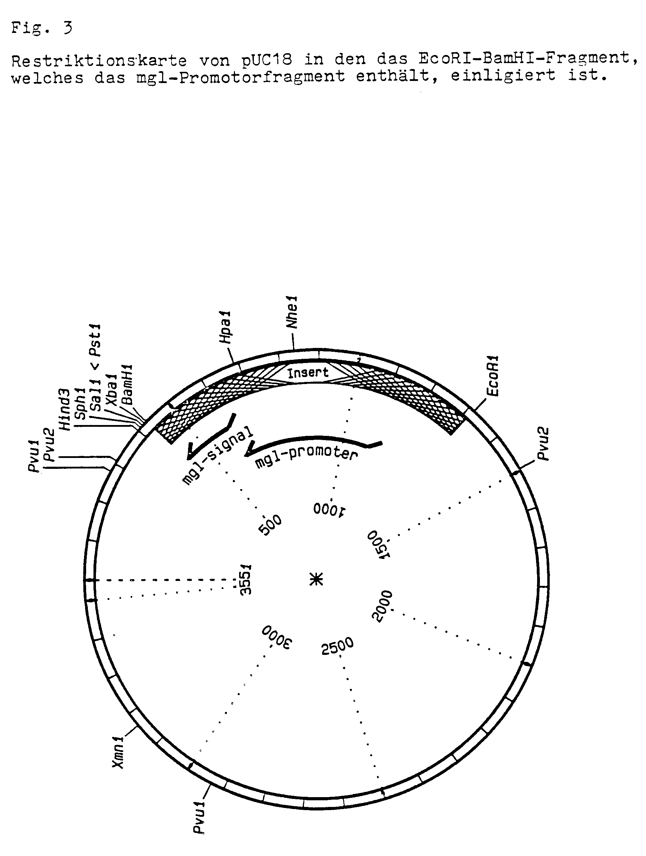

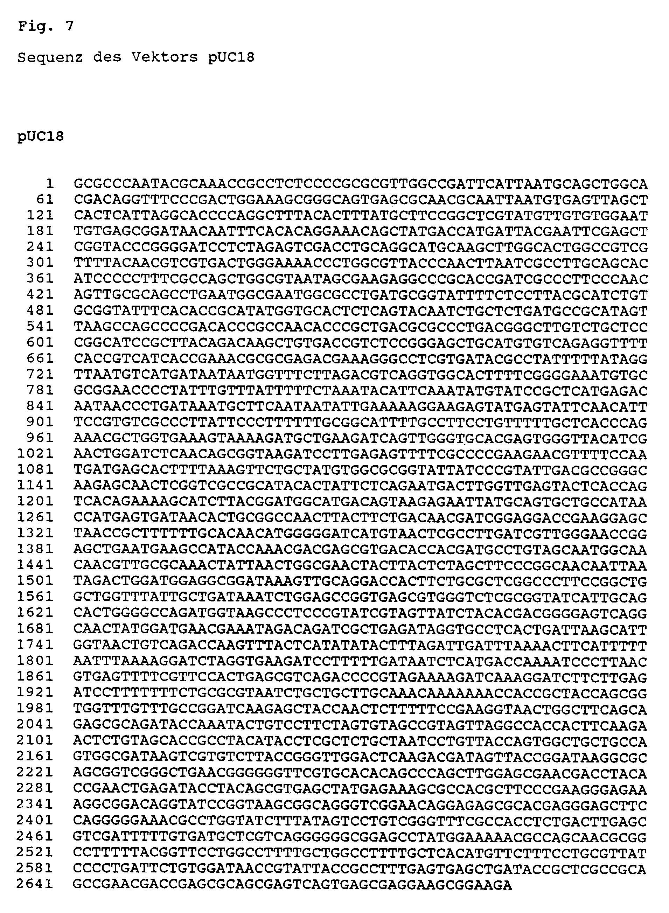

In analoger Weise wird ein gleichwertiges Plasmid hergestellt, in dem das Plasmid pUC18 (Fig. 7) ebenfalls mit EcoRI und BamHI geschnitten wird und dort das genannte Fragment eingesetzt wird (Fig. 3).

Diese beiden Vektoren dienen als Quelle für das DNA-Fragment mit mgl-Promotor und gegebenenfalls zusätzlich mit mgl-B-Signalsequenz zur Konstruktion von Vektoren die unter Kontrolle des mgl-Promotors Fremdgene exprimieren können.

### Beispiel 2

### Konstruktion eines Universal-Vektors mit mgl-Promotor, in den Fremdgene eingebaut werden können.

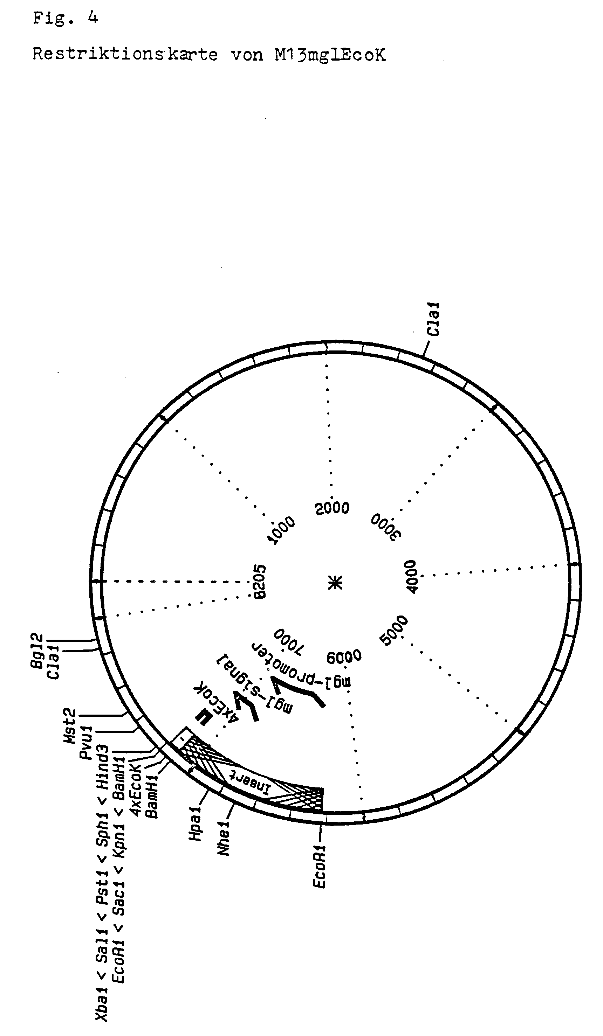

Ein DNA-Fragment aus M13K11, das vier EcoK-Schnittstellen und an den Enden eine BamHI- und eine EcoRI-Schnittstelle trägt (4x EcoK-Cassette) (beschrieben in Nucleic Acids Research 13, (1985), 8561-71) wird zusammen mit dem EcoRI, BamHI-Fragment aus pNM506 (Beispiel 1) in einen mit EcoRI gespaltenen Vektor M13mp18 ligiert. Hierbei entsteht der Vektor M13mglEcoK (Fig. 4). Dieser Vektor besitzt eine Polylinkerregion mit den Schnittstellen KpnI, SacI, HindIII, SphI, PstI, SalI und XbaI, in die beliebige Fremdgene eingesetzt werden können.

### Beispiel 3

### Vektor zur Expression von Endo-β-N-acetylglucosaminidase H (Endo H)

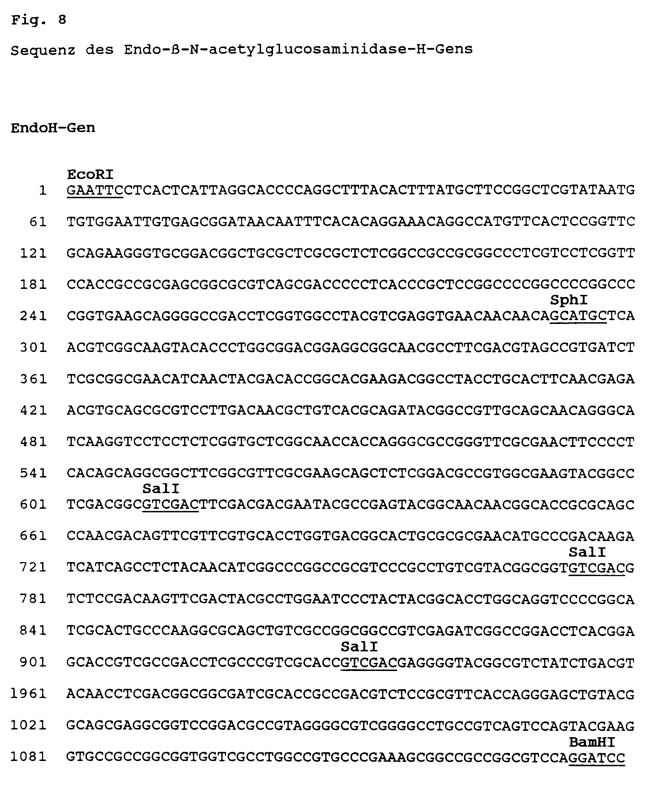

Der durch die Schnittstellen EcoRI und SalI definierte N-terminale Teil des Endo H-Gens (Fig. 8, Länge 609 bp) (Journal of Biological Chemistry 259, (1984) 7577-7583) wird aus dem Plasmid pEH 7' isoliert.

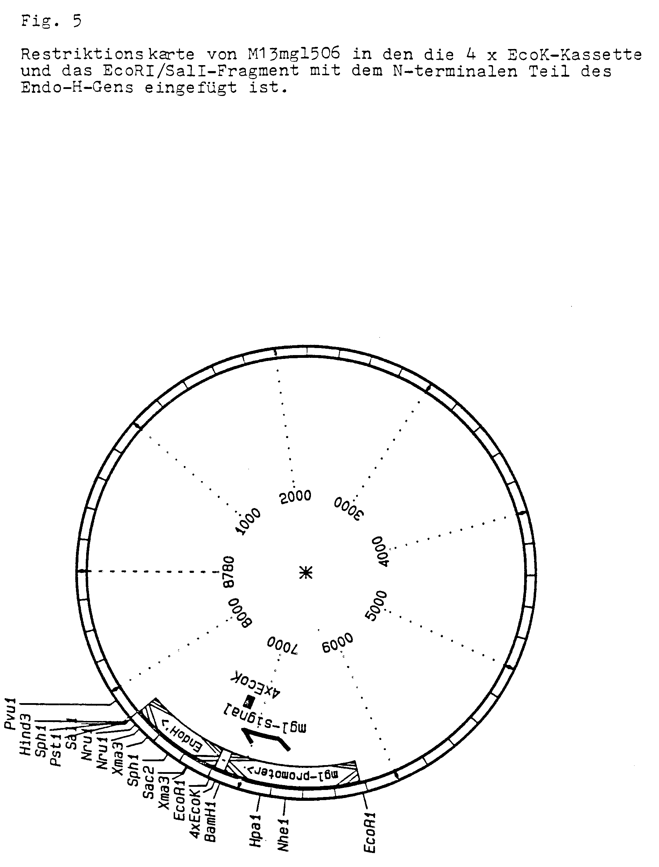

Das aus M13mgl506 (Beispiel 1) durch Schnitt mit BamHI und SalI erhaltene Fragment, das oben beschriebene EcoRI-SalI-Fragment sowie die in Beispiel 2 beschriebene 4x Ecok-Cassette (als EcoRI-BamHI-Fragment) werden mit T4 Ligase ligiert, wobei die Konstruktion gemäß Fig. 5 entsteht.

Durch in vitro Mutagenese (Methods Enzymol 100 (1983), 468-500; Nucleic Acids Res. 10 (1982), 6487-6500) mit einem synthetischen Oligonukleotid der Sequenz
5' CCCCTGCTTC ACCGGGGCCA TGGTAGCTCC GGTTTT 3'
erhält man eine Fusion zwischen dem ATG des mgl-Promotors und dem N-terminalen Teil des reifen EndoH-Gens. Hierbei ist weder eine Signalsequenz von mgl noch von EndoH enthalten. Die Klone, welche die gewünschte Deletion enthalten, werden über ein Screening mit dem oben beschriebenen radioaktiv markierten Oligonukleotid als Probe identifiziert. Von einem der durch dieses Screening identifizierten Klone wird die replikative DNA präpariert.

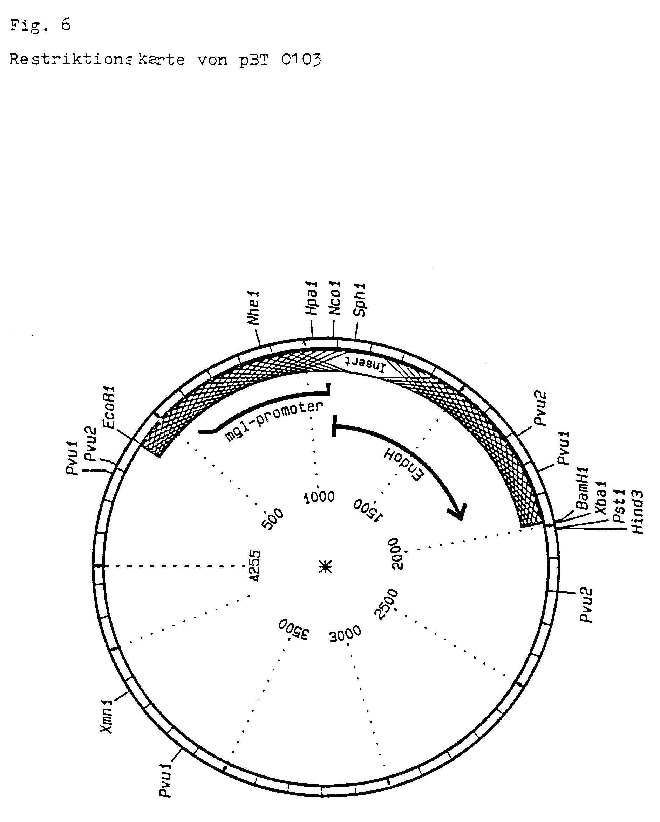

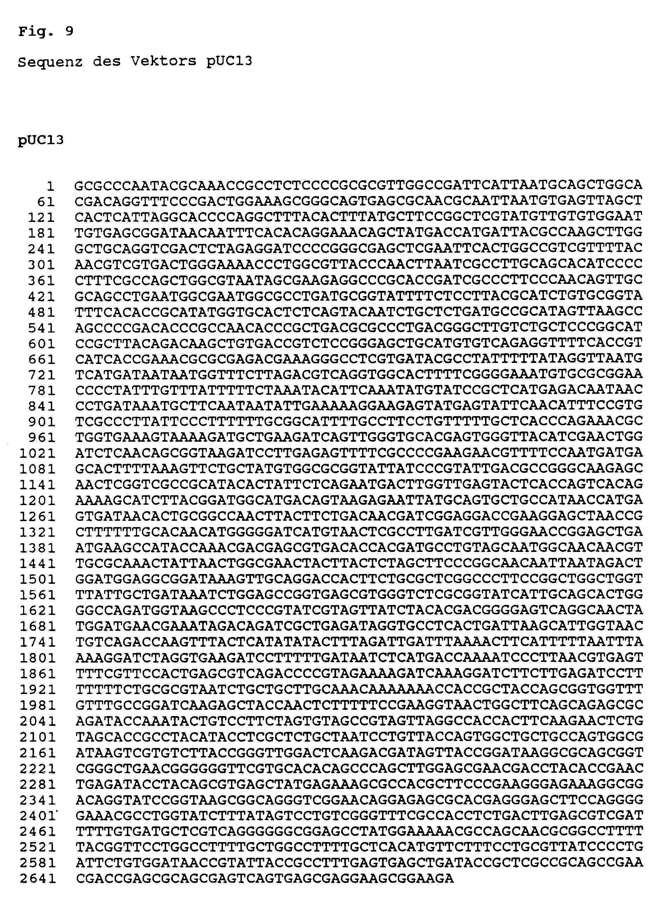

Diese DNA wird mit EcoRI und SphI gespalten. Das hierbei entstehende Fragment (750 Basenpaare) wird mit einem SphI-BamHI-Fragment, welches den Rest des Endo H-Gens von SphI bis über das Ende des Gens (J. Biol. Chem. 259 (1984) 7577-7583) enthält, in einen mit EcoRI und BamHI gespaltenen PUC13-Vektor (Sequenz Fig. 9), ligiert. Hierbei entsteht ein Vektor, der das mgl-Promotor-Gen sowie das vollständige Gen von EndoH trägt (Fig. 6). Dieser Vektor wird mit pBT0103, bezeichnet.

Mit Hilfe dieses Plasmids kann Endoglycosidase H in E. coli HB101, DSM 1607, exprimiert werden. Die Expression läßt sich durch Zugabe von Glucose steuern.

**Tabelle I**

| Anzucht von E. coli HB101 mit Plasmid pBT0103 in LB-Medium (10 g Trypton, 5 g Hefeextrakt, 5 g NaCl Pro Liter) mit und ohne Glucose. | |
|---|---|
| Glucose | Aktivität EndoH (U/l Medium) |
| 0 % | ca. 100 |
| 0,1 % | ca. 10 |
| 0,2 % | 0 |
| 0,4 % | 0 |

Analoge Ergebnisse werden erhalten, wenn anstelle von Glucose Glucose-6-phosphat verwendet wird.

### Beispiel 4

### Vektor mit mgl-Promotor, mgl-Signalsequenz und Endo H-Gen

Das Plasmid pBT0103 (Beispiel 3) wird mit NcoI geschnitten und ein synthetischer Linker, der für das mgl-Signalpeptid kodiert (Fig. 1, Pos. 688-756) durch Ligation eingefügt, so daß ein korrektes Leseraster, sowie korrekte Prozessierung gewährleistet ist.

### Beispiel 5

### Vektor mit mgl-Promotor und β-Galactosidase-Gen ohne Signalsequenz.

Das Plasmid pBT0103 (Beispiel 3) wird mit NcoI/PstI gespalten und das hierbei entstehende 3,3 kb große Fragment isoliert.

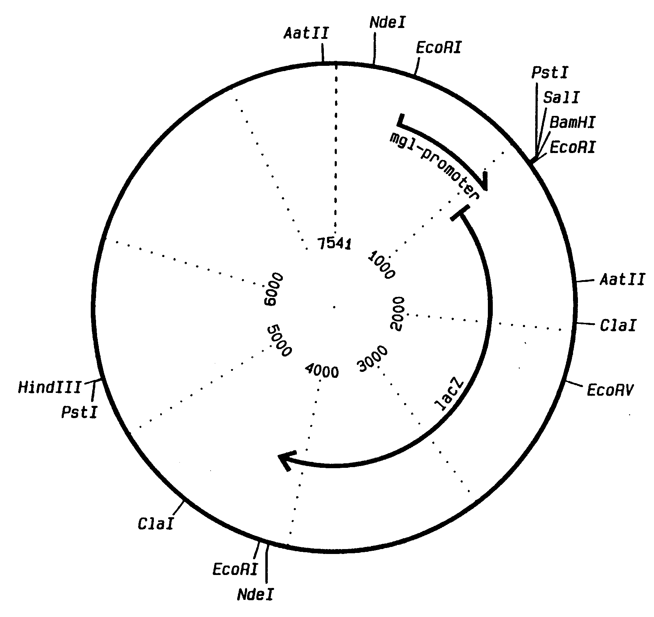

Ein BamHI/PstI-Fragment von ca. 5 kb des Plasmids pBT 117, DSM 3063 (beschrieben in EP 0 180 225 A2), welches den größten Teil des lacZ-Gens enthält, wird präpariert und unter Verwendung eines synthetischen Linkers der Sequenz 
5' C ATG GTT ACG GAT TGC TGC AGG TCG ACG 3'
||| ||| ||| ||| ||| ||| ||| ||
3' CAA TGC CTA ACG ACG TCC AGC TGC CTA G 5'
NcoI PstI SalI BamHI
mit dem 3,3 kb-Fragment ligiert, wobei ein Plasmid erhalten wird, bei dem der Leserahmen des lacZ an das ATG des mgl-Gens fusioniert ist (pPZ07-mgllac, Fig. 10). Bei Einbringen dieses Plasmids in E. coli HB101, DSM 1607, wird eine Expression von β-Galactosidase in die cytoplasmatische Fraktion der Zelle beobachtet, die Glucose-steuerbar ist (Tabelle II).

**Tabelle II**

| Stamm: | β-Galactosidase-Aktivität: | |
|---|---|---|
| | LB-Medium | LB-Medium + 0,2 % Glucose |
| HB101 | 4900 | 4700 |
| HB101 x pPZ07-mgllac | 31400 | 1700 |

(Die β-Galactosidase-Aktivitätsbestimmung wurde wie bei J.H. Miller (1972) Experiments in molecular genetics, Cold Spring Harbor Press, Cold Spring Harbor, New York beschrieben durchgeführt.)

### Beispiel 6

### Expression der Pen-G-Amidase

Das mgl-Expressionsplasmid wurde verwendet, um das periplasmatische Enzym Penicillin G-Amidase zu exprimieren. Die Subklonierung und die DNA-Sequenz der PenG-Amidase sind in EP 0180225 A2 beschrieben. Die repli-kative Form des Phagen M13mgl506 (Beispiel 1) wurde mit HindIII und BamHI gespalten. Die HindIII-Schnittstelle wurde vor der Spaltung mit BamHI mit Polymerase I (Klenow-Fragment) und den 4 Desoxyribonukleotidtriphosphaten glatt gemacht.

Aus dem Plasmid pBT212, DSM3058 (beschrieben in EP 0 180 225 A2), wurde durch Spaltung mit BamHI und AhaIII ein Fragment von ca. 800 bp isoliert. Dieses Fragment wird in den vorher beschriebenen und mit BamHI und HindIII geöffneten Vektor M13mgl506 ligiert. Unter Verwendung des Oligonukleotids
5' ACTTGACGACTGCTCCGCGTGCGCGTGCGC 3'
und der einzelsträngigen DNA des Phagen M13 kann nun eine Deletionsmutagenese durchgeführt werden. Einzelheiten dieser Methode sind im Handbuch "Oligonukleotid-directed in vitro mutagenesis system" Amersham rpn 2322 beschrieben. Durch die Deletion entsteht eine exakte Fusion zwischen der mgl-Signalsequenz und dem Gen der Pen-G-Amidase in der Art, daß in dem Protein, welches aus der DNA übersetzt wird, Aminosäure 23 des mgl-Signalpeptids (Fig. 1) mit Aminosäure 27 der Pen-G-Amidase fusioniert ist. Die Klone, welche die gewünschte Deletion enthalten, werden über ein Screening mit dem oben beschriebenen, radioaktiv markierten Oligonukleotid als Probe identifiziert (vgl. Handbuch loc. cit). Von einem der durch das Screening identifizierten Klone wird die replikative DNA präpariert (vgl. Handbook loc. cit. dort M13 cloning and sequencing).

Die DNA wird mit EcoRI und EcoRV gespalten und das ca. 1 kb große DNA-Fragment isoliert. Dieses DNA-Fragment wird in den mit EcoRI und EcoRV gespaltenen Vektor pBT212 ligiert und damit die kodierende Region der Pen-G-Amidase unter die Regulationskontrolle des mgl-Promotors gebracht.

### Beispiel 7

### Expression und Sekretion Penicillin G Amidase (PenG) über mgl-Promotor und mgl-Signalpeptid

Das Plasmid pBTE1-11 (EP 0180225 A2, DSM 3061) enthält das PenG-Gen. Durch Spaltung mit den Restriktionsendonukleasen ClaI und SphI kann ein 182 bp großes DNA-Fragment erhalten werden, das den N-terminalen Bereich des PenG-Gens einschließlich des Starts der reifen PenG umfaßt. Dieses Fragment wird in AccI-SphI gespaltenen Vektor M13mglEcoK (Fig. 4) einligiert. Durch In-vitro-Mutagenese, wie in Beispiel 3 beschrieben, mit einem synthetischen Oligonukleotid der Sequenz:
1 ACTTGACGAC TGCTCCGCGT GCGCGTG 27
erhält man eine Fusion zwischen dem Ende der mglB-Signalsequenz und dem Start der reifen PenG. Die für das Signalpeptid der PenG kodierende Sequenz fehlt. Die Klone, welche die gewünschte Deletion enthalten, werden über ein Screening mit dem oben beschriebenen radioaktiv markierten Oligonukleotid als Hybridisierungssonde identifiziert. Von einem dieser Klone wird replikative, doppelsträngige DNA präpariert.

Diese DNA wird mit EcoRI und SphI gespalten. Das entstehende DNA-Fragment (812 bp) wird in EcoRI und SphI gespaltenes Vektorplasmid pUC18 einligiert. Nach Transformation von E.coli HB101 wird aus einem korrekten Klon Plasmid-DNA präpariert. Diese DNA wird mit SphI und HindIII gespalten und mit einem DNA-Fragment von 3000 bp Größe ligiert, das aus pBTE1-11 durch Spaltung mit SphI und HindIII gewonnen wurde und den fehlenden C-terminalen Bereich des PenG-Gens enthält.

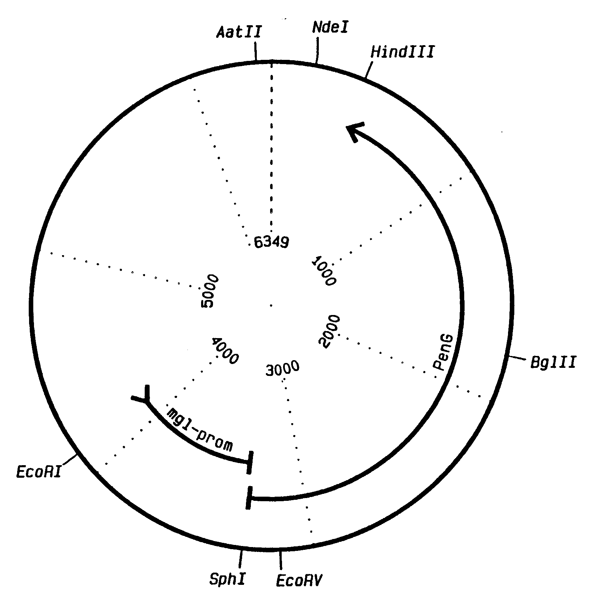

Das dabei entstehende Plasmid, pPZ07-mglpenG (Fig. 11) enthält den für die reife PenG kodierenden Genabschnitt fusioniert an die mglB-Signalsequenz. Die Expression dieses Fusionsgens wird über den mgl-Promotor gesteuert und ist glukoseabhängig (Tabelle III). Exprimierte PenG wird über das mglB-Signalpeptid in den periplasmatischen Raum ausgeschleust und dort zum aktiven Enzym prozessiert.

**Tabelle III**

| Stamm: | PenG Aktivität (mU/A 420 Zelldichte): | |
|---|---|---|
| | LB-Medium | LB-Medium + 0,4% Glucose |
| HB101 x pUC18 | 0 | n.b. |
| HB101 x pPZ07-mglpenG | 20,9 | 0,8 |
| HB101 x pBT E1-11 | 1,7 | n.b. |

(Die PenG-Enzymaktivität wurde mit 2-Nitro-5-phenylacetaminophenylessigsäure als Farbsubstrat, wie bei Kutzbach, C. und Rauenbusch, E. (1974), Hoppe-Seyler's Physiol. Chem. Bd. 354, 45-53 beschrieben, bestimmt.)
Zur Entfernung des in Plasmid pBT212 vorhandenen tac Promotors wird das Plasmid mit BamHI und EcoRI gespalten, die Enden mit DNA-Polymerase (Klenow-Fragment) und den 4 Desoxyribonukleotidphosphaten glatt gemacht und religiert.

Die Expression der Pen-G-Amidase unterliegt in diesem Plasmid der Katabolit-reprimierbaren Kontrolle und wird mit Hilfe der mgl-B-Signalsequenz in das Periplasma ausgeschleust.

## Patentansprüche

1. Expressionsvektor zur regulierbaren Expression von Fremdgenen in Prokaryonten,
**dadurch gekennzeichnet**,
daß er aus einem mit Glukose und anderen Katabolyt-reprimierenden Zuckern nahezu vollständig reprimierbaren DNA-Vektor besteht, der die Regulationssequenz des mgl-Operons, die aus den Nukleotiden 1 bis 704 der DNA-Sequenz, die in Fig. 1 dargestellt ist, oder einer bei Standardbedingungen damit hybridisierenden, aber noch den gesamten mgl-Promotor enthaltenden Sequenz besteht, und die Initiationsstelle der Translation des mgl-Operons sowie wenigstens ein Fremdgen enthält, welches unter der Expressionskontrolle der mgl-Operon-Regulationssequenz steht, wobei sich zwischen dem Promotor und der Insertionsstelle des Fremdgens keine für das reife mglB-Protein codierenden Sequenzen befinden.

2. Expressionsvektor nach Anspruch 1,
**dadurch gekennzeichnet**,
daß der DNA-Vektor ein plasmid oder ein Phagengenom ist.

3. Expressionsvektor nach Anspruch 1 oder 2,
**dadurch gekennzeichnet**,
daß der DNA-Vektor ein Shuttle-Vektor ist, der für grampositive sowie gramnegative Bakterien geeignet ist.

4. Expressionsvektor nach einem der vorhergehenden Ansprüche,
**dadurch gekennzeichnet**,
daß er einen Polylinker enthält, in den das Fremdgen insertiert vorliegt.

5. Expressionsvektor nach einem der vorhergehenden Ansprüche,
**dadurch gekennzeichnet**,
daß er zwischen der regulatorischen mgl-Operonteilsequenz und dem Fremdgen bzw. dem Polylinker eine Signalsequenz enthält, die die räumliche Lokalisation des exprimierten Genprodukts in der Wirtszelle bestimmt.

6. Expressionsvektor nach Anspruch 5,
**dadurch gekennzeichnet**,
daß die Signalsequenz aus der für das signalpeptid des mgl-B-Proteins kodierenden Sequenz besteht.

7. Expressionsvektor nach Anspruch 5,
**dadurch gekennzeichnet**,
daß die mgl-B-Signalsequenz durch eine Konsensussequenz von signalpeptiden ersetzt ist.

8. Plasmid M13mgl1506, dessen Restriktionskarte in Fig. 2 dargestellt ist.

9. Plasmid M13mglEcoK, dessen Restriktionskarte in Fig. 4 dargestellt ist.

10. Verfahren zur Herstellung eines Expressionsvektors nach einem der vorhergehenden Ansprüche,
**dadurch gekennzeichnet**,
daß man die gewünschten Teile des mgl-Operons aus dem Genom einer Zelle, die imstande ist, von außen Zugeführte Galactose zu verwerten, durch Spaltung mit geeigneten Restriktionsenzymen herausschneidet und diese in einen geeigneten DNA-Vektor insertiert.

11. Verfahren nach Anspruch 10,
**dadurch gekennzeichnet**,
daß man die gewünschten Teile des mgl-Operons aus dem Genom von Salmonella typhimurium, DSM 554 oder aus E.coli, DSM 4090 herausschneidet.

12. Verfahren nach Anspruch 10,
**dadurch gekennzeichnet**,
daß man eine DNA-Sequenz, die dem Teil des mgl-Operons aus dem Plasmid pNM506, wie in Fig. 1 dargestellt, entspricht, in einen geeigneten Vektor insertiert.

13. Verfahren nach Anspruch 10 bis 12,
**dadurch gekennzeichnet**,
daß man als DNA-Vektor die replikative Form des Phagen M13mp18 oder einen pUC-Vektor verwendet.

14. Verfahren nach einem der Ansprüche 10 bis 13,
**dadurch gekennzeichnet**,
daß man zusätzlich noch hinter die mgl-Sequenzen einen Polylinker insertiert.

15. Verfahren nach einem der Ansprüche 12 bis 14,
**dadurch gekennzeichnet**,
daß man eine DNA-Sequenz verwendet, die der in Fig. 1 dargestellten Sequenz des Plasmids pNM506 entspricht, welche außer den mgl-Operon-Regulationssequenzen auch die für die Signalsequenz des mgl-B-Proteins codierende Sequenz enthält.

16. Verfahren nach einem der Ansprüche 10 bis 14,
**dadurch gekennzeichnet**,
daß man hinter die mgl-Operonteilsequenz eine für eine Signalsequenz kodierende DNA-Sequenz insertiert.

17. Verfahren nach einem der Ansprüche 10 bis 14,
**dadurch gekennzeichnet**,
daß man hinter die mgl-Operonsequenz eine für eine Konsensus-Sequenz von Signalpeptiden kodierende DNA-Sequenz insertiert.

18. Verfahren zur Herstellung des Plasmids M13mgl1506 (Fig. 2) nach Anspruch 15,
**dadurch gekennzeichnet**,
daß man eine dem EcoRI/BamHI-Fragment des Plasmids pNM506 (Fig. 1) entsprechende DNA-Sequenz in die ebenfalls mit EcoRI und BamHI gespaltene doppelsträngige replikative Form des Phagen M13mp18 einligiert.

19. Verfahren zur Herstellung des Plasmids M13mglEcoK (Fig. 4) nach Anspruch 15,
**dadurch gekennzeichnet**,
daß man eine dem BamHI/EcoRI-Fragment des Plasmids M13K11 entsprechende DNA-Sequenz, die vier EcoK Schnittstellen enthält (4x EcoK-Cassette) zusammen mit einer dem EcoRI/BamHI-Fragment des Plasmids pNM506 (Fig. 1) entsprechenden DNA-Sequenz in die mit EcoRI gespaltene replikative Form des Phagen M13mp18 einligiert.

20. Verwendung eines Expressionsvektors nach den Ansprüchen 1 bis 9 zur regulierbaren Expression eines Fremdgens in Prokaryonten unter der Expressionskontrolle des mgl-Promotors, wobei die Expression des Fremdgens über den Gehalt an Katabolyt-reprimierenden Zuckern im Medium reguliert wird.

21. Verwendung eines Expressionsvektors nach den Ansprüchen 5 bis 9,
**dadurch gekennzeichnet**,
daß die gewünschte Lokalisation des entstandenen Genprodukts in der Zelle durch eine Signalsequenz bestimmt wird.

22. Verwendung nach Anspruch 20 oder 21,
**dadurch gekennzeichnet**,
daß zur Expression ein Prokaryont verwendet wird, der das hergestellte Genprodukt aus dem Periplasma ins Medium abgeben kann.

23. Verwendung nach einem der Ansprüche 20 bis 22,
**dadurch gekennzeichnet**,
daß das hergestellte Genprodukt ein für die Wirtszelle toxisches Protein ist.

24. Verfahren zur Herstellung eines rekombinanten Proteins,
**dadurch gekennzeichnet**,
daß man zur Expression des für das rekombinante Protein kodierenden Gens einen mit Glukose und anderen Katabolyt-reprimierenden Zuckern nahezu vollständig reprimierbaren Expressionsvektor nach einem der Ansprüche 1 bis 7 verwendet.

25. Verfahren nach Anspruch 24,
**dadurch gekennzeichnet**,
daß man ein für die Wirtszelle toxisches rekombinantes Protein herstellt.

## Claims

1. Expression vector for the adjustable expression of foreign genes in prokaryotes, characterised in that it consists of a DNA vector almost completely repressable with glucose and other catabolyte-repressing sugars, of the regulation sequence of the mgl operon which consists of the nucleotides 1 to 704 of the DNA sequence which is illustrated in Fig. 1 or consists of a sequence hybridising therewith under standard conditions but still containing the whole mgl promotor and contains the initiation position of the translation of the mgl operon, as well as at least one foreign gene which stands under the expression control of the mgl operon regulation sequence, whereby, between the promotor and the insertion position of the foreign gene, there are present no sequences coding for the mature mglB protein.

2. Expression vector according to claim 1, characterised in that the DNA vector is a plasmid or a phage genome.

3. Expression vector according to claim 1 or 2, characterised in that the DNA vector is a shuttle vector which is suitable for gram-positive as well as gram-negative bacteria.

4. Expression vector according to one of the preceding claims, characterised in that it contains a polylinker in which the foreign gene is present inserted.

5. Expression vector according to one of the preceding claims, characterised in that, between the regulatory mgl operon partial sequence and the foreign gene or the polylinker, it contains a signal sequence which determines the spatial localisation of the expressed gene product in the host cell.

6. Expression vector according to claim 5, characterised in that the signal sequence consists of the sequence coding for the signal peptide of the mglB protein.

7. Expression vector according to claim 5, characterised in that the mgl B signal sequence is replaced by a consensus sequence of signal peptides.

8. Plasmid M13mgl506, the restriction card of which is illustrated in Fig. 2.

9. Plasmid M13mglEcoK, the restriction card of which is illustrated in Fig. 4.

10. Process for the production of expression vector according to one of the preceding claims, characterised in that one cuts out the desired parts of the mgl operon from the genome of a cell which is able to utilise galactose provided externally by cleavage with suitable restriction enzymes and inserts these into a suitable DNA vector.

11. Process according to claim 10, characterised in that one cuts out the desired parts of the mgl operon from the genome of Salmonella typhimurium, DSM 554, or from E. coli, DSM 4090.

12. Process according to claim 10, characterised in that one inserts a DNA sequence which corresponds to the part of the mgl operon from the plasmid pNM506, as is illustrated in Fig. 1, into a suitable vector.

13. Process according to claims 10 to 12, characterised in that, as DNA vector, one uses the replicative form of the phage M13mp18 or a pUC vector.

14. Process according to one of claims 10 to 13, characterised in that one additionally inserts a polylinker after the mgl sequences.

15. Process according to one of claims 12 to 14, characterised in that one uses a DNA sequence which corresponds to the sequence of the plasmid pNM506 illustrated in Fig. 1 which, apart from the mgl operon regulation sequences, also contains the sequence coding for the signal sequence of the mgl B protein.

16. Process according to one of claims 10 to 14, characterised in that, after the mgl operon signal sequence, one inserts a DNA sequence coding for a signal sequence.

17. Process according to one of claims 10 to 14, characterised in that, after the mgl operon sequence, one inserts a DNA sequence coding for a consensus sequence of signal peptides.

18. Process according to claim 15 for the production of the plasmid M13mgl506 (Fig. 2), characterised in that one ligates a DNA sequence corresponding to the EcoRI/BamHI fragment of the plasmid pNM506 (Fig. 1) into the double-stranded replicative form of the phage M13mp18 also cleaved with EcoRI and BamHI.

19. Process according to claim 15 for the production of the plasmid M13mglEcoK (Fig. 4), characterised in that one ligates a DNA sequence corresponding to the BamHI/EcoRI fragment of the plasmid M13K11 which contains four EcoK cleavage positions (4x EcoK cassette), together with a DNA sequence corresponding to the EcoRI/BamHI fragment of the plasmid pNM506 (Fig. 1), into the replicative form of the phage M13mp18 cleaved with EcoRI.

20. Use of an expression vector according to claims 1 to 9 for the adjustable expression of a foreign gene in prokaryotes under the expression control of the mgl promotor, whereby the expression of the foreign gene is regulated via the content of catabolyte-repressing sugars in the medium.

21. Use of an expression vector according to claims 5 to 9, characterised in that the desired localisation of the resultant gene product in the cell is determined by a signal sequence.

22. Use according to claim 20 or 21, characterised in that, for the expression, a prokaryote is used which can give up the gene product produced from the periplasm into the medium.

23. Use according to one of claims 20 to 22, characterised in that the gene product produced is a toxic protein for the host cell.

24. Process for the production of a recombinant protein, characterised in that, for the expression of the gene coding for the recombinant protein, one uses an expression vector according to one of claims 1 to 7 which is almost completely repressable with glucose or other catabolyte-repressing sugars.

25. Process according to claim 24, characterised in that one produces a recombinant protein toxic for the host cell.

## Revendications

1. Vecteur d'expression pour l'expression régulable de gène étrangers dans des procaryotes, caractérisé en ce qu'il consiste en un vecteur d'ADN sensiblement totalement réprimable par le glucose et d'autres sucres réprimant les catabolytes, qui contient la séquence de régulation de l'opéron mgl qui consiste en les nucléotides 1 à 704 de la séquence d'ADN qui est représentée sur la figure 1 ou en une séquence qui s'hybride avec elle dans des conditions standard mais qui contient encore la totalité du promoteur mgl, et le site d'initiation de la traduction de l'opéron mgl ainsi qu'au moins un gène étranger qui est sous le contrôle d'expression de la séquence de régulation de l'opéron mgl, aucune séquence codant la protéine mgl-B mature ne se trouvant entre le promoteur et le site d'insertion du gène étranger.

2. Vecteur d'expression selon la revendication 1, caractérisé en ce que le vecteur d'ADN est un plasmide ou un génome de phage.

3. Vecteur d'expression selon la revendication 1 ou 2, caractérisé en ce que le vecteur d'ADN est un vecteur navette qui convient pour des bactéries Gram positives et pour des bactéries Gram négatives.

4. Vecteur d'expression selon l'une des revendications précédentes, caractérisé en ce qu'il contient un lieur multisite dans lequel le gène étranger se trouve inséré.

5. Vecteur d'expression selon l'une des revendications précédentes, caractérisé en ce qu'il contient entre la séquence partielle de régulation de l'opéron mgl et le gène étranger ou le lieur multisite une séquence signal qui détermine la localisation spatiale du produit génique exprimé dans la cellule hôte.

6. Vecteur d'expression selon la revendication 5, caractérisé en ce que la séquence signal consiste en la séquence codant le peptide signal de la protéine mgl-B.

7. Vecteur d'expression selon la revendication 5, caractérisé en ce que la séquence signal de mgl-B est remplacée par une séquence consensus de peptides signaux.

8. Plasmide M13mgl1506 dont la carte de restriction est représentée sur la figure 2.

9. Plasmide M13mglEcoK dont la carte de restriction est représentée sur la figure 4.

10. Procédé de préparation d'un vecteur d'expression selon l'une des revendications précédentes, caractérisé en ce que l'on retire les parties voulues de l'opéron mgl du génome d'une cellule qui est en mesure d'utiliser le galactose apporté de l'extérieur, par clivage avec des enzymes de restriction appropriées et on les insère dans un vecteur d'ADN approprié.

11. Procédé selon la revendication 10, caractérisé en ce que l'on retire les parties voulues de l'opéron mgl du génome de Salmonella typhimurium, DSM 554 ou de E. coli, DSM 4090.

12. Procédé selon la revendication 10, caractérisé en ce que l'on insère dans un vecteur approprié une séquence d'ADN qui correspond à la partie de l'opéron mgl provenant du plasmide pNM506 telle que représentée sur la figure 1.

13. Procédé selon les revendications 10 à 12, caractérisé en ce que l'on utilise comme vecteur d'ADN la forme réplicative du phage M13mp18 ou un vecteur pUC.

14. Procédé selon l'une des revendications 10 à 13, caractérisé en ce que l'on insère en outre derrière les séquences mgl un lieur multisite.

15. Procédé selon l'une des revendications 12 à 14, caractérisé en ce que l'on utilise une séquence d'ADN qui correspond à la séquence du plasmide pNM506 représentée sur la figure 1, qui, outre les séquences de régulation de l'opéron mgl, contient aussi la séquence codant la séquence signal de la protéine mgl-B.

16. Procédé selon l'une des revendications 10 à 14, caractérisé en ce que l'on insère derrière la séquence partielle de l'opéron mgl une séquence d'ADN codant une séquence signal.

17. Procédé selon l'une des revendications 10 à 14, caractérisé en ce que l'on insère derrière la séquence de l'opéron mgl une séquence d'ADN codant une séquence consensus de peptides signaux.

18. Procédé de préparation du plasmide M13mgl1506 (figure 2) selon la revendication 15, caractérisé en ce que l'on ligature une séquence d'ADN correspondant au fragment EcoRI/BamHI du plasmide pNM506 (figure 1) dans la forme réplicative double brin du phage M13mp18 elle aussi coupée avec EcoRI et BamHI.

19. Procédé de préparation du plasmide M13mglEcok (figure 4) selon la revendication 15, caractérisé en ce que l'on ligature une séquence d'ADN correspondant au fragment BamHI/EcoRI du plasmide M13K11 qui contient quatre sites de clivage EcoK (cassette 4 x EcoK) en même temps qu'une séquence d'ADN correspondant au fragment EcoRI/BamHI du plasmide pNM506 (figure 1) dans la forme réplicative du phage M13mp18 coupée avec EcoRI.

20. Utilisation d'un vecteur d'expression selon les revendications 1 à 9 pour l'expression régulable d'un gène étranger dans des procaryotes sous le contrôle d'expression du promoteur mgl, l'expression du gène étranger étant régulée par le biais de la teneur du milieu en sucres réprimant les catabolytes.

21. Utilisation d'un vecteur d'expression selon les revendications 5 à 9, caractérisée en ce que la localisation voulue du produit génique formé dans la cellule est déterminée par une séquence signal.

22. Utilisation selon la revendication 20 ou 21, caractérisée en ce que l'on utilise pour l'expression un procaryote qui peut délivrer du périplasme dans le milieu le produit génique formé.

23. Utilisation selon l'une des revendications 20 à 22, caractérisée en ce que le produit génique formé est une protéine toxique pour la cellule hôte.

24. Procédé de préparation d'une protéine recombinante, caractérisé en ce que l'on utilise pour l'expression du gène codant la protéine recombinante un vecteur d'expression sensiblement totalement réprimable par le glucose et d'autres sucres réprimant les catabolytes selon l'une des revendications 1 à 7.

25. Procédé selon la revendication 24, caractérisé en ce que l'on prépare une protéine recombinante toxique pour la cellule hôte.
